# EUROPEAN PATENT APPLICATION

(11) **EP 4 215 222 A1**
(43) Date of publication of application: **26.07.2023**
(21) Application number: 21889527.4
(22) Date of filing: 02.11.2021
(51) Int. Cl.: A61L 27/04, A61L 27/58, A61F 2/30, A61L 31/02, A61L 31/14, C22C 23/04, B22D 21/04

(54) **HIGH-STRENGTH, HIGH-ANTICORROSIVE, BIODEGRADABLE MAGNESIUM ALLOY AND IMPLANT USING SAME**

(30) Priority: 04.11.2020 KR 20200146064; 01.10.2021 KR 20210131125
(71) Applicant: KOREA INSTITUTE OF MATERIALS SCIENCE, Changwon-si, Gyeongsangnam-do 51508 (KR)
(72) Inventor: SUH, Joung-sik, Changwon-si Gyeongsangnam-do 51475 (KR); SUH, Byeongchan, Changwon-si Gyeongsangnam-do 51431 (KR); KIM, Young-min, Changwon-si Gyeongsangnam-do 51382 (KR); KIM, Ha-sik, Changwon-si Gyeongsangnam-do 51204 (KR); MOON, Byung-gi, Changwon-si Gyeongsangnam-do 51170 (KR); MOON, Younghoon, Changwon-si Gyeongsangnam-do 51511 (KR); BAE, Jun-ho, Gimhae-si Gyeongsangnam-do 50998 (KR); YOU, Bong-sun, Busan 49229 (KR); LEE, Sang-eun, Changwon-si Gyeongsangnam-do 51511 (KR); YIM, Changdong, Changwon-si Gyeongsangnam-do 51506 (KR)
(74) Representative: Potter Clarkson
(86) International application number: PCT/KR2021/015729
(87) International publication number: WO 2022/098062

(57) **Abstract**

The present disclosure relates to a biodegradable Mg alloy and a biodegradable implant using the Mg alloy, the Mg alloy comprising; Zn in the amount of at least 0.5 wt.% and less than 3.0 wt.%; Mn in the amount of at least 0.0 wt.% and at most 2.0 wt.%; Sr in the amount of at least 0.0 wt.% and less than 3.0 wt.%; Ca in the amount of at least 0.0 wt.% and at most 2.0 wt.%; and Mg in the rest amount, based on the total weight of the Mg alloy. It is advantageous that the strength and corrosion resistance of the biodegradable Mg alloy can be simultaneously improved according to the above configuration.

## Description

### Technical Field

The present invention relates to biodegradable Mg alloys with high strength and high corrosion resistance and implants using the same.

### Background Art

A medical implant, prosthesis or implant (inclusively referred to as an implant hereinafter) denotes to material inserted or implanted into a human body for treatment purposes. Representative materials for implants include metals, ceramics, and polymers.

In addition, an implant may be categorized as a long-term implant or a short-term implant based on the period of use inside a human body. While the long-term implants are used semi-permanently after implantation inside the body, the short-term implants are inserted and used for a certain period for complete treatment, and removed through a removal surgery in completion of treatment.

When a conventional short-term implant is used, it has been problematic in various ways not only that complicated removal surgery after a certain period of implantation causes patient's severe pain and consumes time and budget but also that side effects may be caused by the secondary removal surgery.

There has been a need to develop a biodegradable implant, as an alternative short-term implant, which degrades within a body after a certain period of implantation so that it does not require secondary removal surgery. Polymer materials, such as polylactic acid, etc., or Mg alloys are being studied as biodegradable materials.

Applications of biodegradable polymers are limited due to their low fracture toughness, acid generation during degradation, difficulty in controlling the biodegradation rate, etc. Meanwhile, due to the very high electrochemical activity of Mg, the corrosion rate of a Mg implant is highly dependent on factors such as implant composition, implantation environment or type of applied area, and the surface conditions of the implant. If a Mg implant is exposed to air, surface of untreated Mg implant reacts with oxygen to deposit a layer of magnesium hydroxide on the surface and eventually delay further chemical reactions. In a human body environment rich in physiological saline media, such untreated Mg implant rapidly corrodes, in the early stages, to produce large amounts of hydrogen gas and magnesium hydroxide. Uncontrolled corrosion of a Mg implant may cause excessively accelerated degradation due to stress corrosion cracking. In addition, high gas emission rates in early stages may form subcutaneous emphysema.

As the background technology of the present invention, Japan Patent Application No. 5846591 describes an implant made of magnesium or magnesium master alloy that is decomposable inside a human body.

### Detailed Description

### Technical Problem

It is an object of the present invention to provide Mg alloy which may be used as an implant material with biodegradability, high strength and high corrosion resistance.

It is a further object of the present invention to provide a biodegradable implant with improved compressive strength and corrosion resistance (erosion resistance) in the as-cast state.

It is an even further object of the present invention to provide a method of effectively manufacturing a biodegradable implant with improved compressive strength and corrosion resistance in the as-cast state.

### Technical Solution

In one aspect, the biodegradable Mg alloy is provided comprising Zn in the amount of at least 0.5 wt.% and less than 3.0 wt.%; Mn in the amount of at least 0.0 wt.% and at most 2.0 wt.%; Sr in the amount of at least 0.0 wt.% and less than 3.0 wt.%; Ca in the amount of at least 0.0 wt.% and at most 2.0 wt.%; and Mg in the rest amount based on the total weight of the Mg alloy.

In one embodiment, the sum of the amount of Zn and the amount of Ca may be at least 0.5 wt.% and at most 3.0 wt.% based on the total weight of the Mg alloy.

In one embodiment, the amount of Zn may be at least 0.5 wt.% and less than 2.0 wt.% based on the total weight of the Mg alloy.

In one embodiment, the amount of Sr may be at least 0.0 wt.% and at most 2.0 wt.% and the amount of Ca may be at least 0.0 wt.% and less than 2.0 wt.% based on the total weight of the Mg alloy.

In one embodiment, the corrosion resistance of the Mg alloy of the present invention may be improved by suppressing micro-galvanic corrosion between a matrix and impurities or secondary phases and by improving passivation state features of a film formed on the surface of the Mg alloy.

In one embodiment, the compressive strength of the biodegradable Mg alloy of the present invention may be at least 250 MPa in the as-cast state.

In one embodiment, the corrosion rate of the biodegradable Mg alloy of the present invention may be 2.0 mm/y or less in the as-cast state.

In one embodiment, after 8 weeks of immersion in phosphate-buffered saline (PBS) solution, the Mg alloy of the present invention may still have more than 0% of the compressive strength in the as-cast state.

In another aspect, a biodegradable implant consisting of the biodegradable Mg alloy of the present disclosure is provided.

In one embodiment, a biodegradable implant of the present disclosure may be an orthopedic device, a parietal facial device or a cardiovascular device.

In another aspect, a method of manufacturing a biodegradable implant is provided, the method comprising steps of; i) preparing a molten mixture by melting components of; Zn in the amount of at least 0.5 wt.% and less than 3.0 wt.%; Mn in the amount of at least 0.0 wt.% and at most 2.0 wt.%; Sr in the amount of at least 0.0 wt.% and less than 3.0 wt.%; Ca in the amount of at least 0.0 wt.% and at most 2.0 wt.%; and Mg in the rest amount, based on the total weight of the Mg alloy, together; and ii) casting the molten mixture of step i) to obtain an implant.

In one embodiment, in the step i) of the method for manufacturing the biodegradable implant of the present disclosure, the sum of the amount of Zn and the amount of Ca may be at least 0.5 wt.% and at most 3.0 wt.% based on the total weight of the Mg alloy.

In one embodiment, in the step i) of the method for manufacturing the biodegradable implant of the present disclosure, the amount of Sr may be at least 0.0 wt.% and at most 2.0 wt.% and the amount of Ca may be at least 0.0 wt.% and less than 2.0 wt.% based on the total weight of the Mg alloy.

In one embodiment, the compressive strength of the manufactured biodegradable implant of the present disclosure may be at least 250 MPa in the as-cast state.

In one embodiment, the corrosion rate of the manufactured biodegradable implant may be 2.0 mm/y or less in the as-cast state.

### Advantageous Effects

According to one embodiment, Mg alloy, which may be used as an implant material with biodegradability, high strength and high corrosion resistance, may be provided.

According to one embodiment, a biodegradable implant is improved in the aspect of the compressive strength and corrosion resistance in the as-cast state. Specifically, the biodegradable implant in the as-cast state may be provided with the compressive strength (ultimate compressive strength, UCS) of at least 250 MPa and the corrosion rate (CR) of 2.0 mm/y or less.

According to one embodiment, a biodegradable implant with high strength and high corrosion resistance may be effectively manufactured.

Other objects and advantages of the present disclosure are more clearly demonstrated by the Detailed Description, Claims and Figures below.

### Brief Descriptions of Drawings

Fig.1 is a graph illustrating compressive strengths and corrosion rates of the Mg alloys according to inventive examples and comparative examples of the present disclosure.
Fig.2a is a graph illustrating effect of Zn content on compressive strengths and corrosion rates of the Mg alloys.
Fig.2b is a photograph illustrating microstructures of as-cast Mg alloys which demonstrates effect of Zn content on compressive strengths and corrosion rates of the Mg alloys.
Fig.3a is a graph illustrating effect of Mn content on compressive strengths and corrosion rates of the Mg alloys with Zn in the amount of 3.0 wt.%.
Fig.3b is a graph illustrating effect of Mn content on compressive strengths and corrosion rates of the Mg alloy with Zn in the amount of 1.0 wt.%.
Fig.4a is a graph illustrating effect of Sr content on compressive strengths and corrosion rates of the Mg alloys with Zn in the amount of 3.0 wt.%.
Fig.4b is a graph illustrating effect of Sr content on compressive strengths and corrosion rates of the Mg alloys with Zn in the amount of 1.0 wt.%.
Fig.5a is a graph illustrating effect of Ca content on compressive strengths and corrosion rates of the Mg alloys with Zn in the amount of 1.0 wt.%.
Fig.5b is a graph illustrating effect of Ca content on compressive strengths and corrosion rates of the Mg alloys with Zn in the amount of 0.5 wt.%.
Fig.6a is a graph illustrating effect of Zn content on compressive strengths and corrosion rates of the Mg alloys with Ca in the amount of 0.5 wt.%.
Fig.6b is a graph illustrating effect of Zn content on compressive strengths and corrosion rates of the Mg alloys with Ca in the amount of 1.0 wt.%.
Fig.7 is a graph illustrating effect of Mn content on compressive strengths and corrosion rates of the Mg alloys with Ca in the amount of 0.5 wt.%.
Fig.8 is a graph illustrating effect of Sr content on compressive strengths and corrosion rates of the Mg alloys with Ca in the amount of 0.5 wt.%.
Fig.9 is a graph illustrating compressive strengths and corrosion rates of Mg alloys according to the component content of alloys.
Fig.10a is a graph illustrating compressive strengths of Mg alloys according to inventive examples and comparative examples of the present disclosure after 8 weeks of immersion.
Fig.10b is a graph illustrating compressive strengths of Mg alloys according to inventive examples and comparative examples of the present disclosure after 16 weeks of immersion.
Fig.11 is a graph illustrating measurements of thermodynamics-based secondary phase fractions according to the Zn content.
Fig.12 is a graph illustrating measurements of thermodynamics-based secondary phase fractions according to the Mn content.
Figs. 13 to 16 are photographic images illustrating exterior appearance of corrosion specimens after immersion tests of the as-cast Mg alloys according to inventive examples and comparative examples of the present disclosure.
Figs. 17 to 21 are photographic images illustrating microstructures of as-cast Mg alloys according to inventive examples and comparative examples of the present disclosure.

### Best mode

Terms used in the present specification are for explaining embodiments rather than limiting the invention.

Unless otherwise stated, a singular form includes a plural form in this present specification. In this specification, terms such as "include," "comprise," "provide," or "have" should be understood as indicating presence of features, numbers, steps, actions, components, parts or combinations thereof rather than precluding possibilities of presence or addition of one or more other features, numbers steps, actions, components, parts or combinations thereof.

In the present application, when a part "comprises" a component, the description may further comprise other components rather than excluding other components unless stated otherwise. In addition, throughout the entire specification, the term of being "on" a component refers that a component is positioned above or below the target component and does not necessarily mean that a component is positioned above the object based on the gravity direction.

Since the present invention may be variously modified and have several exemplary embodiments, specific exemplary embodiments will be illustrated in the drawings and described in detail in the detailed description. However, it is to be understood that the present disclosure is not limited to the specific exemplary embodiments, but includes all modifications, equivalents and substitutions included in the idea and the technical scope of the present invention. When it is determined that a detailed description for any known art related to the present invention may obscure the gist of the present disclosure, the detailed description will be omitted.

The terms such as "first" and "second" may be used to describe various components and the components are not limited by the above terms. The terms are merely used to distinguish one component from the other component.

Hereinafter, an exemplary embodiment of the present disclosure will be described in detail with reference to the accompanying drawings. In describing an exemplary embodiment of the present invention with reference to the accompanying drawings, components that are the same as or correspond to each other will be denoted by the same reference numerals and an overlapped description thereof will be omitted.

In one aspect, the biodegradable Mg alloy is provided comprising; Zn in the amount of at least 0.5 wt.% and less than 3.0 wt.%; Mn in the amount of at least 0.0 wt.% and at most 2.0 wt.%; Sr in the amount of at least 0.0 wt.% and less than 3.0 wt.%; Ca in the amount of at least 0.0 wt.% and at most 2.0 wt.%; and Mg in the rest amount based on the total weight of the Mg alloy.

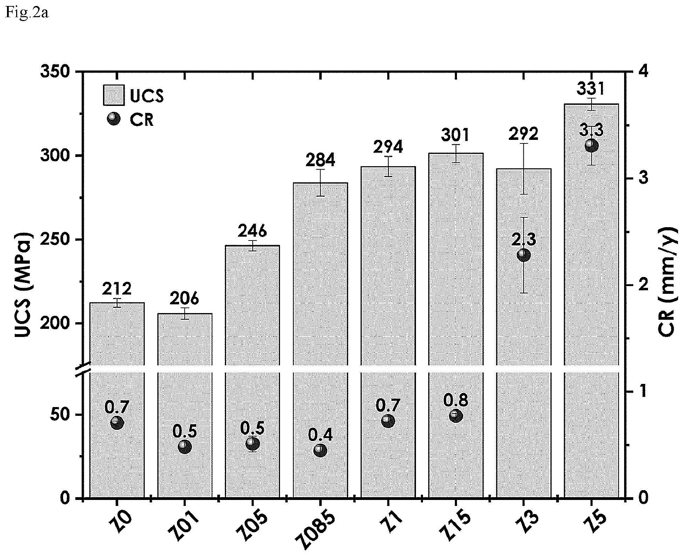

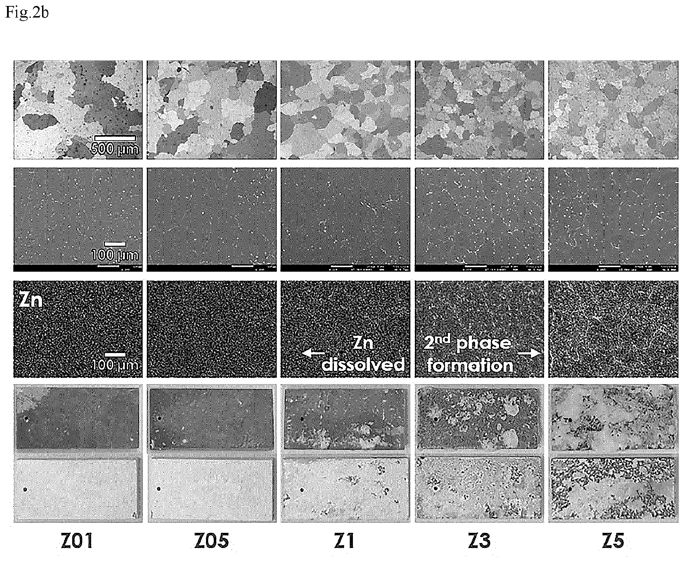

As Zn of the present disclosure is an essential element of a human body, its deficiency may cause disturbances of physiological functions. Zn in the Mg alloy of the present disclosure may contribute to the formation of a passive film to enhance corrosion features and may contribute to the improvement of strength due to solid solution hardening. Although not limited thereto, in order to improve strength and corrosion resistance simultaneously, it may be preferable to comprise Zn in the amount of at least 0.5 wt.% and less than 3.0 wt.%, more preferably of at least 0.5 wt.% and at most 2.0 wt.%, even more preferably of at least 0.5 wt.% and less than 2.0 wt.%, even more preferably of at least 0.5 wt.% and at most 1.5 wt.% and even further more preferably of at least 0.5 wt.% and at most 1.0 wt.% based on the total weight of the Mg alloy. Zn content of 3.0 wt.% and more may dramatically increase the total fraction of the secondary phase (see Fig.2a and Fig.2b).

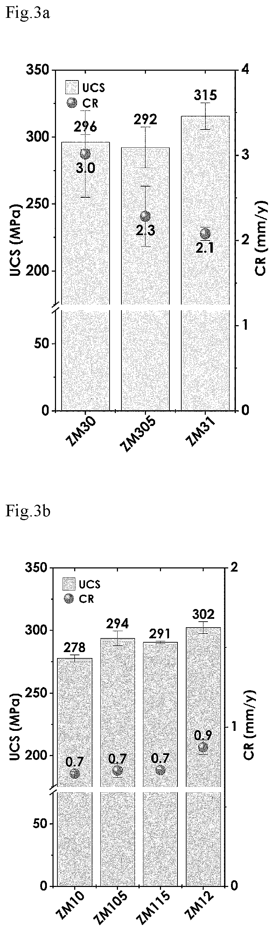

As Mn in the present disclosure is a non-toxic element, which plays a major role in the activation of enzyme systems, it may react with Fe impurities to remove them and contribute to improving the corrosion features of Mg alloys. In addition, it may contribute to improving the strength and elongation of the Mg alloy. Although not limited thereto, in order to improve strength and corrosion resistance simultaneously, it may be preferable to comprise Mn in the amount of at least 0.0 wt.% and at most 2.0 wt.%, more preferably of at least 0.5 wt.% and at most 2.0 wt.% and even more preferably of at least 0.5 wt.% and at most 1.5 wt.% based on the total weight of the Mg alloy (see Fig.3b).

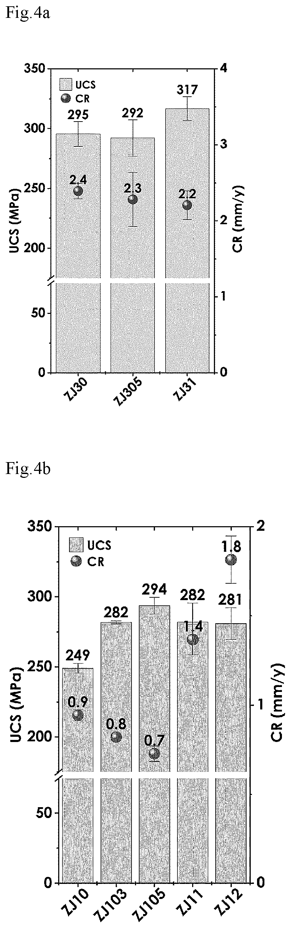

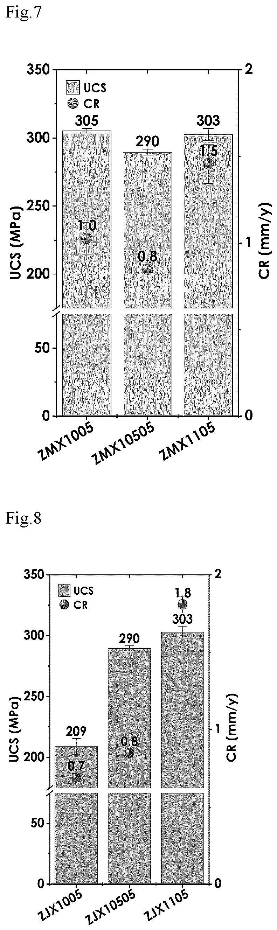

Sr in the present disclosure has an effect similar to that of Ca by functioning as an osteogenesis promoting element to activate osteoblasts and inhibit bone resorption. In addition, it may be effective in improving the strength of Mg alloy through grain refinement. Although not limited thereto, in order to improve strength and corrosion resistance simultaneously, it may be preferable to comprise Sr in the amount of at least 0.0 wt.% and less than 3.0 wt.%, more preferably of at least 0.0 wt.% and at most 2.0 wt.% and even more preferably of at least 0.3 wt.% and at most 2.0 wt.% based on the total weight of the Mg alloy. Specifically, Sr in the amount of at least 0.3 wt.% and less than 1.0 wt.% may be suitable for improvement of strength and corrosion resistance (see Fig.4b and Fig.8).

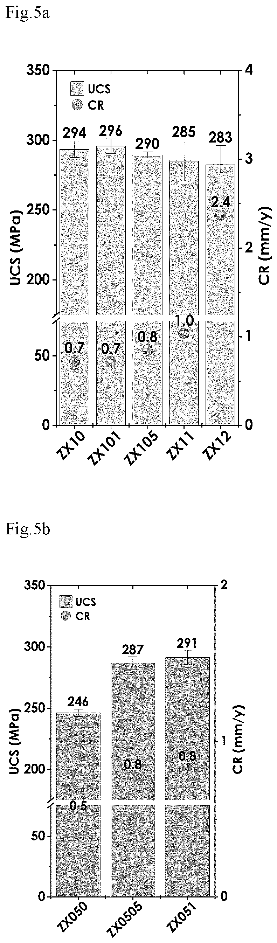

Ca of the present disclosure is an essential element of the human body as a bone forming element. It may induce bone formation and maturation through calcification. In addition, it may be effective in improvement of strength of Mg alloys through grain refinement. Although not limited thereto, in order to improve strength and corrosion resistance simultaneously, it may be preferable to comprise Ca in the amount of at least 0.0 wt.% and at most 2.0 wt.%, more preferably of at least 0.0 wt.% and less than 2.0 wt.%, even more preferably of at least 0.1 wt.% and less than 2.0 wt.% and even further more preferably of at least 0.1 wt.% and at most 1.0 wt.% based on the total weight of the Mg alloy (see Fig. 5a).

Accordingly, every element of Zn, Mn, Sr and Ca in the present disclosure is harmless as they are in-vivo components. By addition of combination of Zn, Mn, Sr and Ca, corrosion resistance of the Mg alloy may be improved by suppressing micro-galvanic corrosion between a matrix and impurities and improving passivation features of a film formed on the surface.

Although not limited thereto, in order to improve strength and corrosion resistance simultaneously, the sum of the amount of Zn and the amount of Ca may preferably be at least 0.5 wt.% and at most 3.0 wt.%, more preferably at least 0.5 wt.% and at most 2.0 wt.% and even more preferably more than 0.5 wt.% and at most 2.0 wt.%.

The compressive strength of the biodegradable Mg alloy of the present disclosure may be at least 250 MPa and at most 305 MPa in the as-cast state. Accordingly, the biodegradable Mg alloy of the present disclosure may satisfy the strength requirement of a biodegradable implant material.

The corrosion rate of the biodegradable Mg alloy in the as-cast state of the present disclosure may preferably be 2.0 mm/y or less, more preferably 1.5 mm/y or less and even more preferably 1.0 mm/y or less. Accordingly, the biodegradable Mg alloy of the present disclosure may be prevented from loss of function due to uncontrolled corrosion of the Mg implant before treatment is complete.

After immersing for 8 weeks in phosphate-buffered saline (PBS) solution, the biodegradable Mg alloy may still have more than 0%, at least 10%, at least 20%, at least 28%, at least 30%, at least 40%, at least 50%, at least 60%, at least 64%, at least 70%, at least 80%, at least 82% or at least 86% of the compressive strength in the as-cast state.

Accordingly, the biodegradable Mg alloy of the present disclosure may prevent the implant inserted in vivo from being degraded too early due to uncontrolled corrosion of the Mg implant.

According to another aspect, a biodegradable implant comprising the biodegradable Mg alloy of the present disclosure is provided. As described above, compressive strength and corrosion resistance of an implant comprising the biodegradable Mg alloy of the present disclosure are improved simultaneously. Specifically, the implant inserted in vivo may be prevented from too early degradation due to uncontrolled corrosion of the Mg implant.

Although not limited thereto, the biodegradable implant of the present disclosure may be suitable for an orthopedic device, a parietal facial device or a cardiovascular device, more suitable for an orthopedic device.

According to another aspect, a method of manufacturing a biodegradable implant is provided, the method comprising the steps of; i) preparing molten mixture by melting components of; Zn in the amount of at least 0.5 wt.% and less than 3.0 wt.%; Mn in the amount of at least 0.0 wt.% and at most 2.0 wt.%; Sr in the amount of at least 0.0 wt.% and less than 3.0 wt.%; Ca in the amount of at least 0.0 wt.% and at most 2.0 wt.%; and Mg in the rest amount, based on the total weight of the Mg alloy, together; and ii) casting the molten mixture of step i) to obtain an implant.

In step i) of the method for manufacturing a biodegradable implant of the present disclosure, the sum of the amount of Zn and the amount of Ca may be at least 0.5 wt.% and at most 3.0 wt.% based on the total weight of the Mg alloy.

In step i) of the method for manufacturing a biodegradable implant of the present disclosure, the amount of Sr may be at least 0.0 wt.% and at most 2.0 wt.% and the amount of Ca may be at least 0.0 wt.% and less than 2.0 wt.% based on the total amount of the Mg alloy.

The compressive strength of the biodegradable implant manufactured by the manufacturing method of the present disclosure may be at least 250 MPa in the as-cast state, which may satisfy the strength requirement as a biodegradable implant material.

The corrosion rate of the biodegradable implant manufactured by the manufacturing method of the present disclosure may be preferably 2.0 mm/y or less, more preferably 1.5 mm/y or less and more preferably 1.0 mm/y or less in the as-cast state. Accordingly, the biodegradable Mg alloy of the present disclosure may be prevented from loss of function due to uncontrolled corrosion of the Mg implant before treatment is complete.

### Examples

### Inventive Examples and Comparative Examples: Preparation of Mg alloy

### Technical Field

The present invention relates to biodegradable Mg alloys with high strength and high corrosion resistance and implants using the same.

### Background Art

A medical implant, prosthesis or implant (inclusively referred to as an implant hereinafter) denotes to material inserted or implanted into a human body for treatment purposes. Representative materials for implants include metals, ceramics, and polymers.

In addition, an implant may be categorized as a long-term implant or a short-term implant based on the period of use inside a human body. While the long-term implants are used semi-permanently after implantation inside the body, the short-term implants are inserted and used for a certain period for complete treatment, and removed through a removal surgery in completion of treatment.

When a conventional short-term implant is used, it has been problematic in various ways not only that complicated removal surgery after a certain period of implantation causes patient's severe pain and consumes time and budget but also that side effects may be caused by the secondary removal surgery.

There has been a need to develop a biodegradable implant, as an alternative short-term implant, which degrades within a body after a certain period of implantation so that it does not require secondary removal surgery. Polymer materials, such as polylactic acid, etc., or Mg alloys are being studied as biodegradable materials.

Applications of biodegradable polymers are limited due to their low fracture toughness, acid generation during degradation, difficulty in controlling the biodegradation rate, etc. Meanwhile, due to the very high electrochemical activity of Mg, the corrosion rate of a Mg implant is highly dependent on factors such as implant composition, implantation environment or type of applied area, and the surface conditions of the implant. If a Mg implant is exposed to air, surface of untreated Mg implant reacts with oxygen to deposit a layer of magnesium hydroxide on the surface and eventually delay further chemical reactions. In a human body environment rich in physiological saline media, such untreated Mg implant rapidly corrodes, in the early stages, to produce large amounts of hydrogen gas and magnesium hydroxide. Uncontrolled corrosion of a Mg implant may cause excessively accelerated degradation due to stress corrosion cracking. In addition, high gas emission rates in early stages may form subcutaneous emphysema.

As the background technology of the present invention, Japan Patent Application No. 5846591 describes an implant made of magnesium or magnesium master alloy that is decomposable inside a human body.

### Detailed Description

### Technical Problem

It is an object of the present invention to provide Mg alloy which may be used as an implant material with biodegradability, high strength and high corrosion resistance.

It is a further object of the present invention to provide a biodegradable implant with improved compressive strength and corrosion resistance (erosion resistance) in the as-cast state.

It is an even further object of the present invention to provide a method of effectively manufacturing a biodegradable implant with improved compressive strength and corrosion resistance in the as-cast state.

### Technical Solution

In one aspect, the biodegradable Mg alloy is provided comprising Zn in the amount of at least 0.5 wt.% and less than 3.0 wt.%; Mn in the amount of at least 0.0 wt.% and at most 2.0 wt.%; Sr in the amount of at least 0.0 wt.% and less than 3.0 wt.%; Ca in the amount of at least 0.0 wt.% and at most 2.0 wt.%; and Mg in the rest amount based on the total weight of the Mg alloy.

In one embodiment, the sum of the amount of Zn and the amount of Ca may be at least 0.5 wt.% and at most 3.0 wt.% based on the total weight of the Mg alloy.

In one embodiment, the amount of Zn may be at least 0.5 wt.% and less than 2.0 wt.% based on the total weight of the Mg alloy.

In one embodiment, the amount of Sr may be at least 0.0 wt.% and at most 2.0 wt.% and the amount of Ca may be at least 0.0 wt.% and less than 2.0 wt.% based on the total weight of the Mg alloy.

In one embodiment, the corrosion resistance of the Mg alloy of the present invention may be improved by suppressing micro-galvanic corrosion between a matrix and impurities or secondary phases and by improving passivation state features of a film formed on the surface of the Mg alloy.

In one embodiment, the compressive strength of the biodegradable Mg alloy of the present invention may be at least 250 MPa in the as-cast state.

In one embodiment, the corrosion rate of the biodegradable Mg alloy of the present invention may be 2.0 mm/y or less in the as-cast state.

In one embodiment, after 8 weeks of immersion in phosphate-buffered saline (PBS) solution, the Mg alloy of the present invention may still have more than 0% of the compressive strength in the as-cast state.

In another aspect, a biodegradable implant consisting of the biodegradable Mg alloy of the present disclosure is provided.

In one embodiment, a biodegradable implant of the present disclosure may be an orthopedic device, a parietal facial device or a cardiovascular device.

In another aspect, a method of manufacturing a biodegradable implant is provided, the method comprising steps of; i) preparing a molten mixture by melting components of; Zn in the amount of at least 0.5 wt.% and less than 3.0 wt.%; Mn in the amount of at least 0.0 wt.% and at most 2.0 wt.%; Sr in the amount of at least 0.0 wt.% and less than 3.0 wt.%; Ca in the amount of at least 0.0 wt.% and at most 2.0 wt.%; and Mg in the rest amount, based on the total weight of the Mg alloy, together; and ii) casting the molten mixture of step i) to obtain an implant.

In one embodiment, in the step i) of the method for manufacturing the biodegradable implant of the present disclosure, the sum of the amount of Zn and the amount of Ca may be at least 0.5 wt.% and at most 3.0 wt.% based on the total weight of the Mg alloy.

In one embodiment, in the step i) of the method for manufacturing the biodegradable implant of the present disclosure, the amount of Sr may be at least 0.0 wt.% and at most 2.0 wt.% and the amount of Ca may be at least 0.0 wt.% and less than 2.0 wt.% based on the total weight of the Mg alloy.

In one embodiment, the compressive strength of the manufactured biodegradable implant of the present disclosure may be at least 250 MPa in the as-cast state.

In one embodiment, the corrosion rate of the manufactured biodegradable implant may be 2.0 mm/y or less in the as-cast state.

### Advantageous Effects

According to one embodiment, Mg alloy, which may be used as an implant material with biodegradability, high strength and high corrosion resistance, may be provided.

According to one embodiment, a biodegradable implant is improved in the aspect of the compressive strength and corrosion resistance in the as-cast state. Specifically, the biodegradable implant in the as-cast state may be provided with the compressive strength (ultimate compressive strength, UCS) of at least 250 MPa and the corrosion rate (CR) of 2.0 mm/y or less.

According to one embodiment, a biodegradable implant with high strength and high corrosion resistance may be effectively manufactured.

Other objects and advantages of the present disclosure are more clearly demonstrated by the Detailed Description, Claims and Figures below.

### Brief Descriptions of Drawings

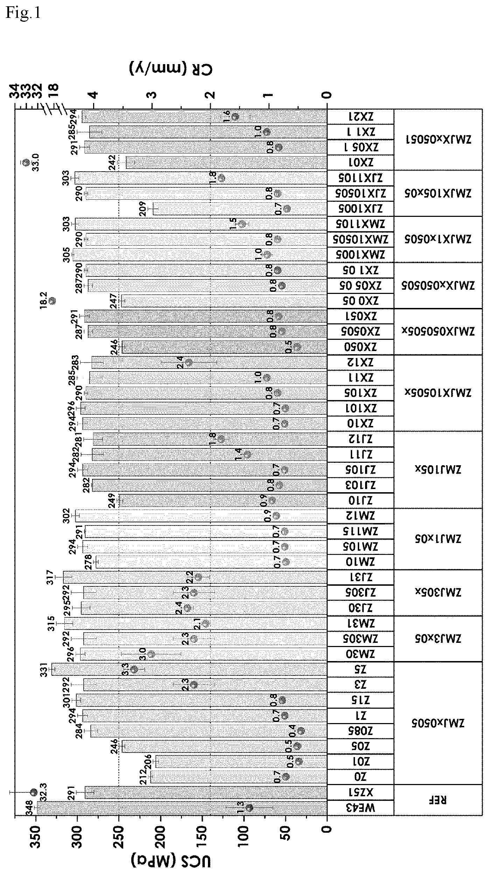

Fig.1 is a graph illustrating compressive strengths and corrosion rates of the Mg alloys according to inventive examples and comparative examples of the present disclosure.
Fig.2a is a graph illustrating effect of Zn content on compressive strengths and corrosion rates of the Mg alloys.
Fig.2b is a photograph illustrating microstructures of as-cast Mg alloys which demonstrates effect of Zn content on compressive strengths and corrosion rates of the Mg alloys.
Fig.3a is a graph illustrating effect of Mn content on compressive strengths and corrosion rates of the Mg alloys with Zn in the amount of 3.0 wt.%.
Fig.3b is a graph illustrating effect of Mn content on compressive strengths and corrosion rates of the Mg alloy with Zn in the amount of 1.0 wt.%.
Fig.4a is a graph illustrating effect of Sr content on compressive strengths and corrosion rates of the Mg alloys with Zn in the amount of 3.0 wt.%.
Fig.4b is a graph illustrating effect of Sr content on compressive strengths and corrosion rates of the Mg alloys with Zn in the amount of 1.0 wt.%.
Fig.5a is a graph illustrating effect of Ca content on compressive strengths and corrosion rates of the Mg alloys with Zn in the amount of 1.0 wt.%.
Fig.5b is a graph illustrating effect of Ca content on compressive strengths and corrosion rates of the Mg alloys with Zn in the amount of 0.5 wt.%.

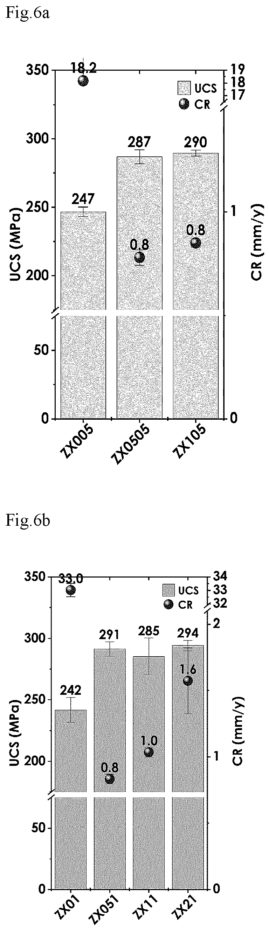

Fig.6a is a graph illustrating effect of Zn content on compressive strengths and corrosion rates of the Mg alloys with Ca in the amount of 0.5 wt.%.
Fig.6b is a graph illustrating effect of Zn content on compressive strengths and corrosion rates of the Mg alloys with Ca in the amount of 1.0 wt.%.
Fig.7 is a graph illustrating effect of Mn content on compressive strengths and corrosion rates of the Mg alloys with Ca in the amount of 0.5 wt.%.
Fig.8 is a graph illustrating effect of Sr content on compressive strengths and corrosion rates of the Mg alloys with Ca in the amount of 0.5 wt.%.

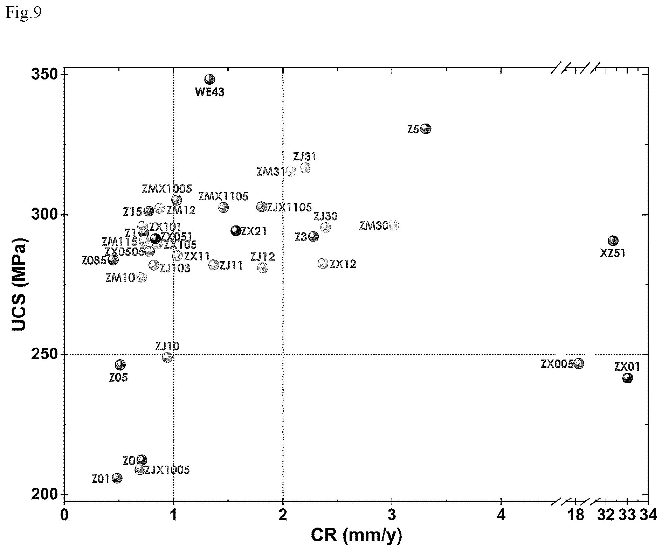

Fig.9 is a graph illustrating compressive strengths and corrosion rates of Mg alloys according to the component content of alloys.

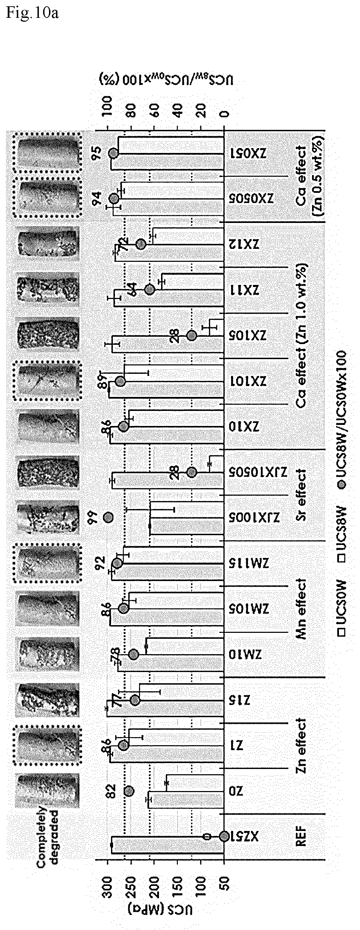

Fig.10a is a graph illustrating compressive strengths of Mg alloys according to inventive examples and comparative examples of the present disclosure after 8 weeks of immersion.

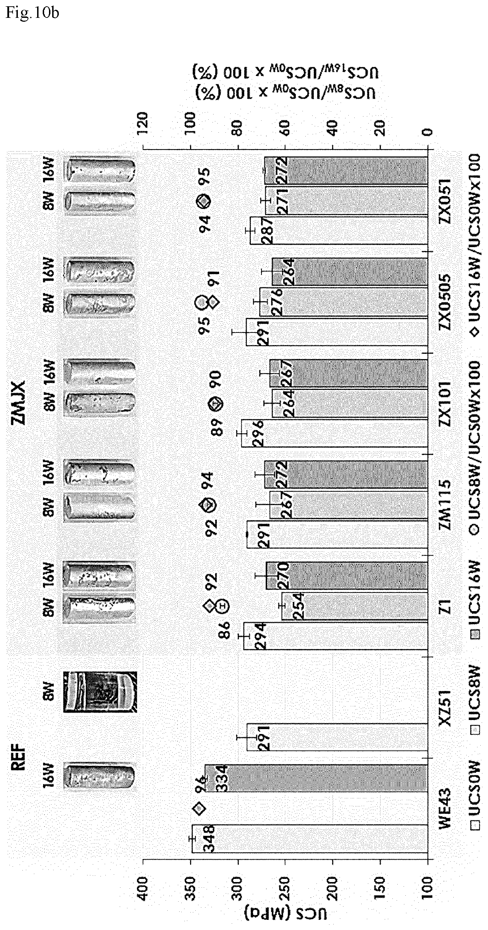

Fig.10b is a graph illustrating compressive strengths of Mg alloys according to inventive examples and comparative examples of the present disclosure after 16 weeks of immersion.

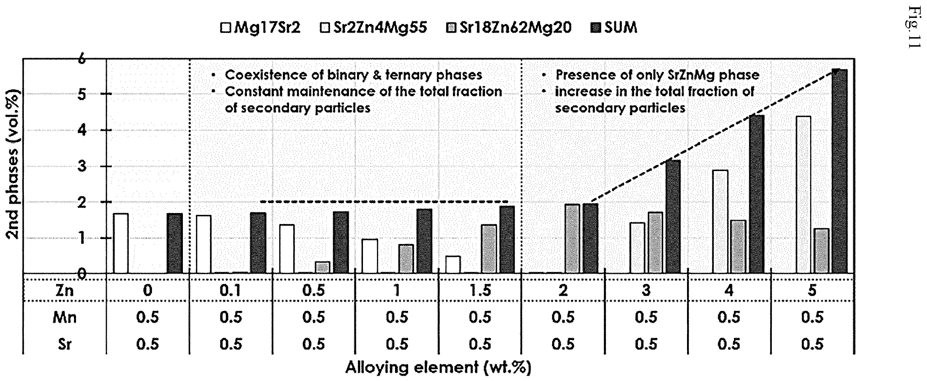

Fig.11 is a graph illustrating measurements of thermodynamics-based secondary phase fractions according to the Zn content.

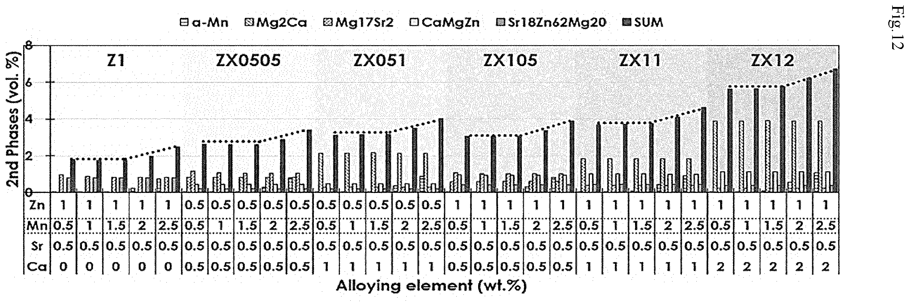

Fig.12 is a graph illustrating measurements of thermodynamics-based secondary phase fractions according to the Mn content.

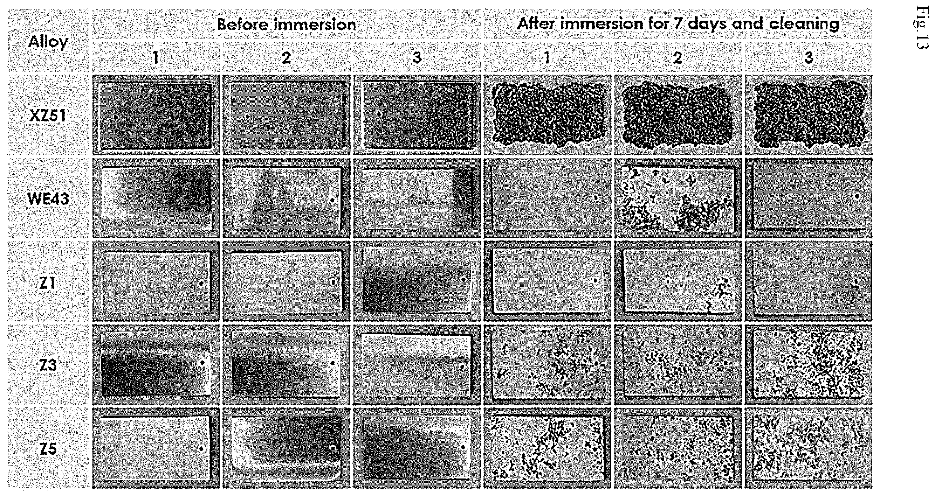

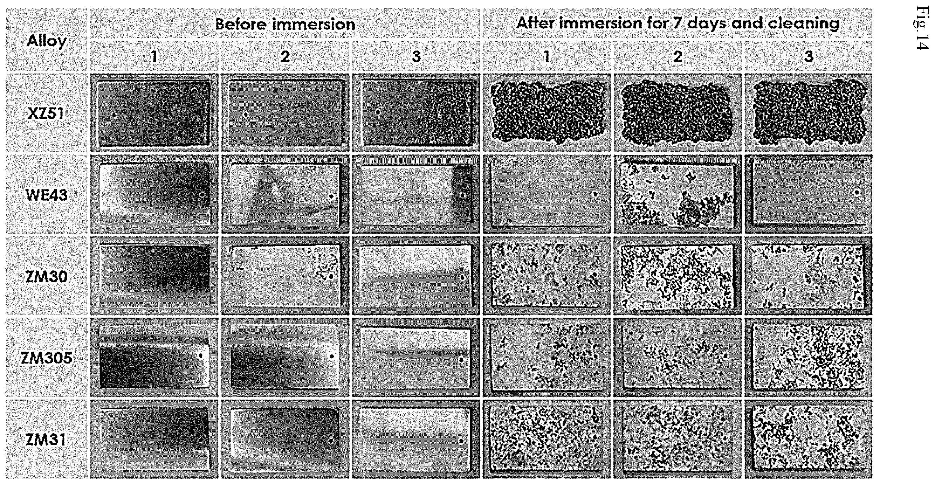

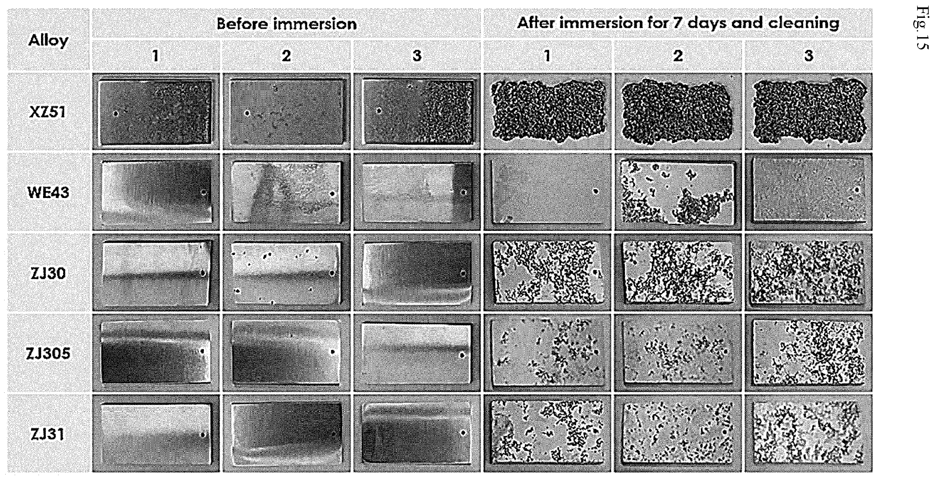

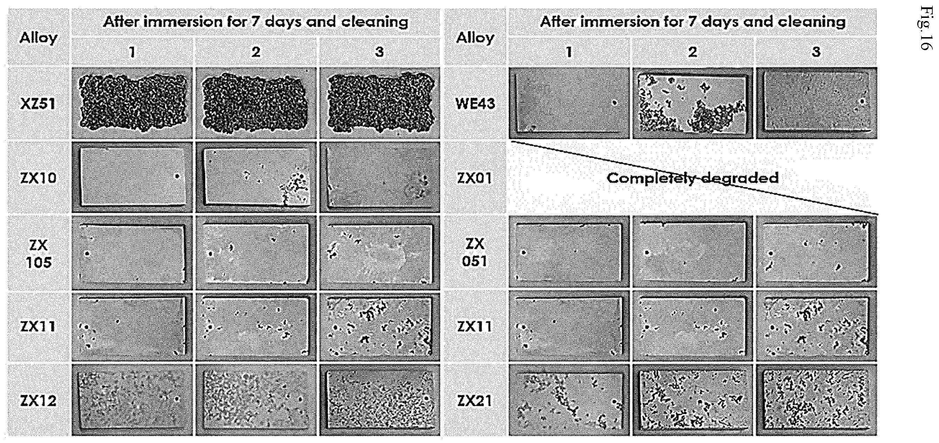

Figs. 13 to 16 are photographic images illustrating exterior appearance of corrosion specimens after immersion tests of the as-cast Mg alloys according to inventive examples and comparative examples of the present disclosure.

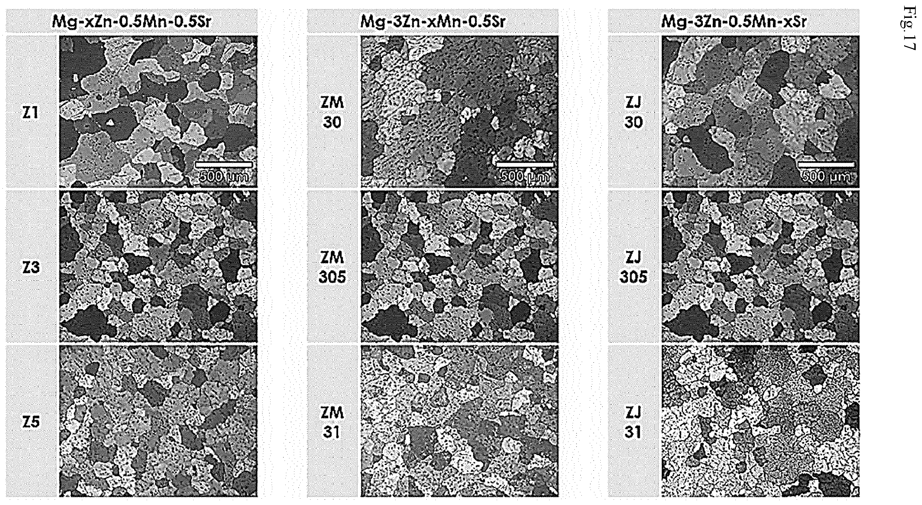

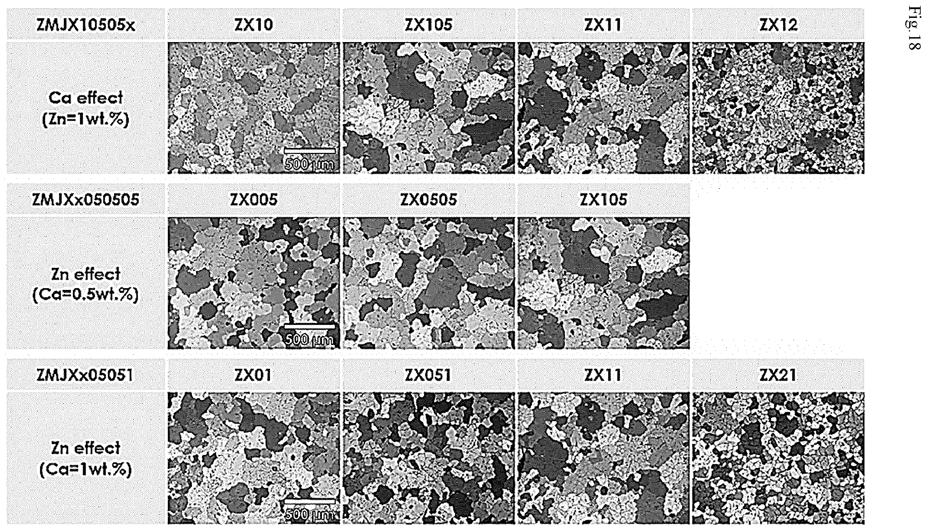

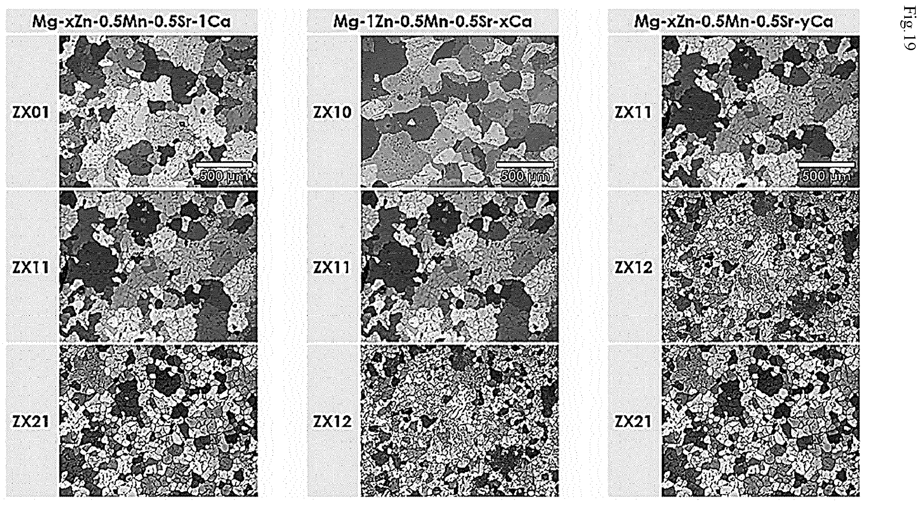

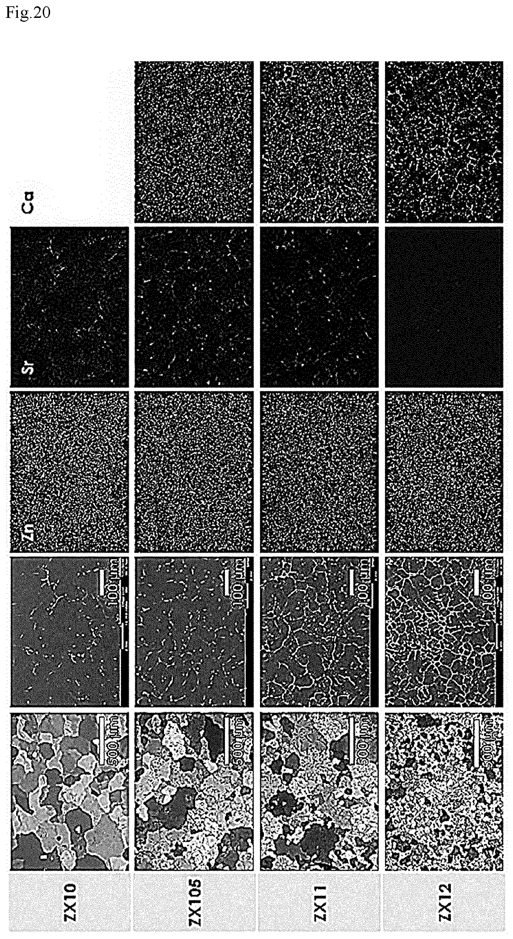

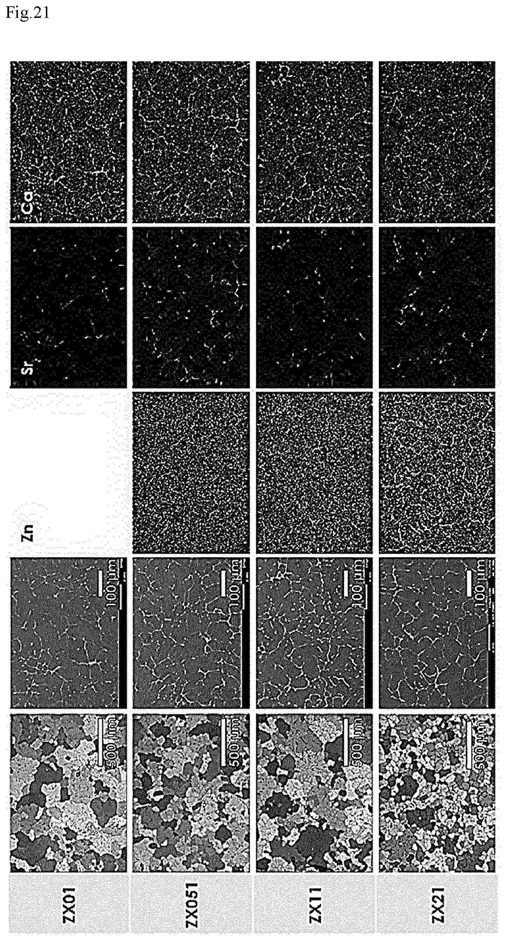

Figs. 17 to 21 are photographic images illustrating microstructures of as-cast Mg alloys according to inventive examples and comparative examples of the present disclosure.

### Best mode

Terms used in the present specification are for explaining embodiments rather than limiting the invention.

Unless otherwise stated, a singular form includes a plural form in this present specification. In this specification, terms such as "include," "comprise," "provide," or "have" should be understood as indicating presence of features, numbers, steps, actions, components, parts or combinations thereof rather than precluding possibilities of presence or addition of one or more other features, numbers steps, actions, components, parts or combinations thereof.

In the present application, when a part "comprises" a component, the description may further comprise other components rather than excluding other components unless stated otherwise. In addition, throughout the entire specification, the term of being "on" a component refers that a component is positioned above or below the target component and does not necessarily mean that a component is positioned above the object based on the gravity direction.

Since the present invention may be variously modified and have several exemplary embodiments, specific exemplary embodiments will be illustrated in the drawings and described in detail in the detailed description. However, it is to be understood that the present disclosure is not limited to the specific exemplary embodiments, but includes all modifications, equivalents and substitutions included in the idea and the technical scope of the present invention. When it is determined that a detailed description for any known art related to the present invention may obscure the gist of the present disclosure, the detailed description will be omitted.

The terms such as "first" and "second" may be used to describe various components and the components are not limited by the above terms. The terms are merely used to distinguish one component from the other component.

Hereinafter, an exemplary embodiment of the present disclosure will be described in detail with reference to the accompanying drawings. In describing an exemplary embodiment of the present invention with reference to the accompanying drawings, components that are the same as or correspond to each other will be denoted by the same reference numerals and an overlapped description thereof will be omitted.

In one aspect, the biodegradable Mg alloy is provided comprising; Zn in the amount of at least 0.5 wt.% and less than 3.0 wt.%; Mn in the amount of at least 0.0 wt.% and at most 2.0 wt.%; Sr in the amount of at least 0.0 wt.% and less than 3.0 wt.%; Ca in the amount of at least 0.0 wt.% and at most 2.0 wt.%; and Mg in the rest amount based on the total weight of the Mg alloy.

As Zn of the present disclosure is an essential element of a human body, its deficiency may cause disturbances of physiological functions. Zn in the Mg alloy of the present disclosure may contribute to the formation of a passive film to enhance corrosion features and may contribute to the improvement of strength due to solid solution hardening. Although not limited thereto, in order to improve strength and corrosion resistance simultaneously, it may be preferable to comprise Zn in the amount of at least 0.5 wt.% and less than 3.0 wt.%, more preferably of at least 0.5 wt.% and at most 2.0 wt.%, even more preferably of at least 0.5 wt.% and less than 2.0 wt.%, even more preferably of at least 0.5 wt.% and at most 1.5 wt.% and even further more preferably of at least 0.5 wt.% and at most 1.0 wt.% based on the total weight of the Mg alloy. Zn content of 3.0 wt.% and more may dramatically increase the total fraction of the secondary phase (see Fig.2a and Fig.2b).

As Mn in the present disclosure is a non-toxic element, which plays a major role in the activation of enzyme systems, it may react with Fe impurities to remove them and contribute to improving the corrosion features of Mg alloys. In addition, it may contribute to improving the strength and elongation of the Mg alloy. Although not limited thereto, in order to improve strength and corrosion resistance simultaneously, it may be preferable to comprise Mn in the amount of at least 0.0 wt.% and at most 2.0 wt.%, more preferably of at least 0.5 wt.% and at most 2.0 wt.% and even more preferably of at least 0.5 wt.% and at most 1.5 wt.% based on the total weight of the Mg alloy (see Fig.3b).

Sr in the present disclosure has an effect similar to that of Ca by functioning as an osteogenesis promoting element to activate osteoblasts and inhibit bone resorption. In addition, it may be effective in improving the strength of Mg alloy through grain refinement. Although not limited thereto, in order to improve strength and corrosion resistance simultaneously, it may be preferable to comprise Sr in the amount of at least 0.0 wt.% and less than 3.0 wt.%, more preferably of at least 0.0 wt.% and at most 2.0 wt.% and even more preferably of at least 0.3 wt.% and at most 2.0 wt.% based on the total weight of the Mg alloy. Specifically, Sr in the amount of at least 0.3 wt.% and less than 1.0 wt.% may be suitable for improvement of strength and corrosion resistance (see Fig.4b and Fig.8).

Ca of the present disclosure is an essential element of the human body as a bone forming element. It may induce bone formation and maturation through calcification. In addition, it may be effective in improvement of strength of Mg alloys through grain refinement. Although not limited thereto, in order to improve strength and corrosion resistance simultaneously, it may be preferable to comprise Ca in the amount of at least 0.0 wt.% and at most 2.0 wt.%, more preferably of at least 0.0 wt.% and less than 2.0 wt.%, even more preferably of at least 0.1 wt.% and less than 2.0 wt.% and even further more preferably of at least 0.1 wt.% and at most 1.0 wt.% based on the total weight of the Mg alloy (see Fig.5a).

Accordingly, every element of Zn, Mn, Sr and Ca in the present disclosure is harmless as they are in-vivo components. By addition of combination of Zn, Mn, Sr and Ca, corrosion resistance of the Mg alloy may be improved by suppressing micro-galvanic corrosion between a matrix and impurities and improving passivation features of a film formed on the surface.

Although not limited thereto, in order to improve strength and corrosion resistance simultaneously, the sum of the amount of Zn and the amount of Ca may preferably be at least 0.5 wt.% and at most 3.0 wt.%, more preferably at least 0.5 wt.% and at most 2.0 wt.% and even more preferably more than 0.5 wt.% and at most 2.0 wt.%.

The compressive strength of the biodegradable Mg alloy of the present disclosure may be at least 250 MPa and at most 305 MPa in the as-cast state. Accordingly, the biodegradable Mg alloy of the present disclosure may satisfy the strength requirement of a biodegradable implant material.

The corrosion rate of the biodegradable Mg alloy in the as-cast state of the present disclosure may preferably be 2.0 mm/y or less, more preferably 1.5 mm/y or less and even more preferably 1.0 mm/y or less. Accordingly, the biodegradable Mg alloy of the present disclosure may be prevented from loss of function due to uncontrolled corrosion of the Mg implant before treatment is complete.

After immersing for 8 weeks in phosphate-buffered saline (PBS) solution, the biodegradable Mg alloy may still have more than 0%, at least 10%, at least 20%, at least 28%, at least 30%, at least 40%, at least 50%, at least 60%, at least 64%, at least 70%, at least 80%, at least 82% or at least 86% of the compressive strength in the as-cast state.

Accordingly, the biodegradable Mg alloy of the present disclosure may prevent the implant inserted in vivo from being degraded too early due to uncontrolled corrosion of the Mg implant.

According to another aspect, a biodegradable implant comprising the biodegradable Mg alloy of the present disclosure is provided. As described above, compressive strength and corrosion resistance of an implant comprising the biodegradable Mg alloy of the present disclosure are improved simultaneously. Specifically, the implant inserted in vivo may be prevented from too early degradation due to uncontrolled corrosion of the Mg implant.

Although not limited thereto, the biodegradable implant of the present disclosure may be suitable for an orthopedic device, a parietal facial device or a cardiovascular device, more suitable for an orthopedic device.

According to another aspect, a method of manufacturing a biodegradable implant is provided, the method comprising the steps of; i) preparing molten mixture by melting components of; Zn in the amount of at least 0.5 wt.% and less than 3.0 wt.%; Mn in the amount of at least 0.0 wt.% and at most 2.0 wt.%; Sr in the amount of at least 0.0 wt.% and less than 3.0 wt.%; Ca in the amount of at least 0.0 wt.% and at most 2.0 wt.%; and Mg in the rest amount, based on the total weight of the Mg alloy, together; and ii) casting the molten mixture of step i) to obtain an implant.

In step i) of the method for manufacturing a biodegradable implant of the present disclosure, the sum of the amount of Zn and the amount of Ca may be at least 0.5 wt.% and at most 3.0 wt.% based on the total weight of the Mg alloy.

In step i) of the method for manufacturing a biodegradable implant of the present disclosure, the amount of Sr may be at least 0.0 wt.% and at most 2.0 wt.% and the amount of Ca may be at least 0.0 wt.% and less than 2.0 wt.% based on the total amount of the Mg alloy.

The compressive strength of the biodegradable implant manufactured by the manufacturing method of the present disclosure may be at least 250 MPa in the as-cast state, which may satisfy the strength requirement as a biodegradable implant material.

The corrosion rate of the biodegradable implant manufactured by the manufacturing method of the present disclosure may be preferably 2.0 mm/y or less, more preferably 1.5 mm/y or less and more preferably 1.0 mm/y or less in the as-cast state. Accordingly, the biodegradable Mg alloy of the present disclosure may be prevented from loss of function due to uncontrolled corrosion of the Mg implant before treatment is complete.

### Examples

### Inventive Examples and Comparative Examples: Preparation of Mg alloy

According to the compositions shown in Tables 1 to 3, melt mixtures of inventive examples 1 to 21 and comparative examples 1 to 13 were prepared and cast.

Casting was performed by dissolving at 760°C, stabilizing at 720°C and subsequently injecting the molten metal at 700°C into a plate-shaped mold preheated at 200°C.

### Experimental Examples: Measurement of Compressive Strength and Corrosion Rate

Compressive strengths and corrosion rates were measured for the as-cast samples manufactured by the Mg alloys of the prepared inventive examples and comparative examples above.

Compression tests at room temperature were performed according to ASTM E9-19 standards and the tests were repeated at least three times with specimens of *ϕ* 13x38 in mm at a strain rate of 10⁻³ s⁻¹.

Corrosion rates were tested according to ASTM G31-72 & G1-03 standards with specimens of 16x32x2 in mm as immersion tests. The immersion tests were performed by immersing in PBS solution for 7 days. The solution was replaced every day and the temperature was set at 36.5°C equivalent to human body temperature. The corrosion rates were measured in

According to the compositions shown in Tables 1 to 3, melt mixtures of inventive examples 1 to 21 and comparative examples 1 to 13 were prepared and cast.

Casting was performed by dissolving at 760°C, stabilizing at 720°C and subsequently injecting the molten metal at 700°C into a plate-shaped mold preheated at 200°C.

### Experimental Examples: Measurement of Compressive Strength and Corrosion Rate

Compressive strengths and corrosion rates were measured for the as-cast samples manufactured by the Mg alloys of the prepared inventive examples and comparative examples above.

Compression tests at room temperature were performed according to ASTM E9-19 standards and the tests were repeated at least three times with specimens of *ϕ* 13x38 in mm at a strain rate of 10⁻³ s⁻¹.

Corrosion rates were tested according to ASTM G31-72 & G1-03 standards with specimens of 16x32x2 in mm as immersion tests. The immersion tests were performed by immersing in PBS solution for 7 days. The solution was replaced every day and the temperature was set at 36.5°C equivalent to human body temperature. The corrosion rates were measured in the amount of weight of the specimens reduced. For the immersion test, three specimens were repeatedly tested per alloy.

To measure compressive strength after immersing for 8 weeks and 16 weeks, cylindrical compression specimens of *ϕ* 13x38 in mm were immersed in PBS solution for 8 weeks. Then, compression tests at room temperature were performed according to ASTM E9-19 standards. The ratios (UCS_{8W}/UCS_{0W} ×100) between initial compressive strength (UCS_{0W}) and compressive strength after 8 weeks (UCSsw) and the ratios (UCS_{16W}/UCS_{0W}×100) between initial compressive strength (UCS_{0W}) and compressive strength after 16 weeks (UCS_{16W}) were measured. PBS solution was not replaced during the 8 week and 16 week of immersions. The temperature was set at 36.5°C, which is equivalent to human body temperature. Three specimens were repeatedly tested per alloy.

Compressive strengths and corrosion rates measured according to the method above are shown in Tables 1 to 3 and Figs. 1 to 12 below.

**[Table 1]**

| | | **Alloy** | | | **Properties** | | **Standard Deviation (SD)** | |
|---|---|---|---|---|---|---|---|---|
| | | **Alloy** | **Chemical Composition (wt.%)** | **Code** | **UCS (MPα)** | **CR (mm/y)** | **UCS (MPα)** | **CR (mm/y)** |
| **Commercial Alloys** | **Comparative Example 01** | **WE43** | **4Y-3RE** | **WO** | **348.2** | **1.33** | **3.4** | **0.40** |
| | **Comparative Example 02** | **XZ51** | **5Cα-1Zn** | **U0** | **290.7** | **32.34** | **10.6** | **2.13** |
| **Zn Effect** | **Comparative Example 03** | **ZMJx0505** | **0Zn-0.5Mn-0.5Sr** | **Z0** | **212.3** | **0.71** | **2.7** | **0.02** |
| | **Comparative Example 04** | | **0.1Zn-0.5Mn-0.5Sr** | **Z01** | **205.9** | **0.48** | **3.4** | **0.05** |
| | **Inventive Example 01** | | **0.5Zn-0.5Mn-0.5Sr** | **Z05** | **246.3** | **0.51** | **3.1** | **0.07** |
| | **Inventive Example 02** | | **0.85Zn-0.5Mn-0.5Sr** | **Z085** | **283.8** | **0.45** | **7.9** | **0.04** |
| **- Mn 0.5 wt.%** | **Inventive Example 03** | | **1Zn-0.5Mn-0.5Sr** | **Z1** | **293.6** | **0.73** | **6.0** | **0.04** |
| **- Sr 0.5 wt.%** | **Inventive Example 04** | | **1.5Zn-0.5Mn-0.5Sr** | **Z15** | **301.2** | **0.77** | **5.4** | **0.02** |
| | **Comparative Example 05** | | **3Zn-0.5Mn-0.5Sr** | **Z3** | **292.2** | **2.28** | **15.1** | **0.36** |
| | **Comparative Example 06** | | **5Zn-0.5Mn-0.5Sr** | **Z5** | **330.7** | **3.31** | **3.6** | **0.18** |
| **Mn Effect** | **Comparative Example 07** | **ZMJ3x05** | **3Zn-0Mn-0.5Sr** | **ZM30** | **296.3** | **3.01** | **5.7** | **0.51** |
| **-Zn 3.0 wt.%** | **Comparative Example 05** | | **3Zn-0.5Mn-0.5Sr** | **ZM305** | **292.2** | **2.28** | **15.1** | **0.36** |
| **- Sr 0.5 wt.%** | **Comparative Example 08** | | **3Zn-1Mn-0.5Sr** | **ZM31** | **315.4** | **2.08** | **9.9** | **0.08** |
| **Sr Effect** | **Comparative Example 09** | **ZMJ305x** | **3Zn-0.5Mn-0Sr** | **ZJ30** | **295.5** | **2.39** | **10.5** | **0.10** |
| **- Zn 3.0 wt.%** | **Comparative Example 05** | | **3Zn-0.5Mn-0.5Sr** | **ZJ305** | **292.2** | **2.28** | **15.1** | **0.36** |
| **- Mn 0.5 wt.%** | **Comparative Example 10** | | **3Zn-0.5Mn-1Sr** | **Z3J1** | **316.7** | **2.21** | **10.1** | **0.19** |

**[Table 2]**

| | | **Alloy** | | | **Properties** | | **Standard Deviation (SD)** | |
|---|---|---|---|---|---|---|---|---|
| | | **Alloy** | **Chemical Composition (wt.%)** | **Code** | **UCS (MPα)** | **CR (mm/y)** | **UCS (MPα)** | **CR (mm/y)** |
| | **Inventive Example 05** | **ZMJ1x05** | **1Zn-0Mn-0.5Sr** | **ZM10** | **277.7** | **0.71** | **2.9** | **0.01** |
| **MnEffect - Zn 1.0wt.%** | **Inventive Example 03** | | **1Zn-0.5Mn-0.5Sr** | **ZM105** | **293.6** | **0.73** | **6.0** | **0.04** |
| **-Sr0.5wt.%** | **Inventive Example 06** | | **1Zn-1Mn-0.5Sr** | **ZM11** | **290.5** | **0.73** | **1.0** | **0.00** |
| | **Inventive Example 07** | | **1Zn-2Mn-0.5Sr** | **ZM12** | **302.4** | **0.87** | **4.7** | **0.05** |
| | **Inventive Example 08** | **ZMJ105X** | **1Zn-0.5Mn-0Sr** | **ZJ10** | **248.9** | **0.94** | **3.5** | **0.03** |
| **Sr Effect** | **Inventive Example 09** | | **1Zn-0.5Mn-0.3Sr** | **ZJ103** | **281.9** | **0.82** | **1.1** | **0.02** |
| **-Zn 1.0 wt.%** | **Inventive Example 03** | | **1Zn-0.5Mn-0.5Sr** | **ZJ105** | **293.6** | **0.73** | **6.0** | **0.04** |
| **- Mn 0.5 wt.%** | **Inventive Example 10** | | **1Zn-0.5Mn-1Sr** | **ZJ11** | **282.0** | **1.37** | **13.5** | **0.09** |
| | **Inventive Example 11** | | **1Zn-0.5Mn-2Sr** | **ZJ12** | **281.0** | **1.82** | **11.3** | **0.13** |
| | **Inventive Example 03** | **ZMJX105050x** | **1Zn-0.5Mn-0.5Sr-0Cα** | **ZX10** | **293.6** | **0.73** | **6.0** | **0.04** |
| **Ca Effect** | **Inventive Example 12** | | **1Zn-0.5Mn-0.5Sr-0.1Ca** | **ZX101** | **295.9** | **0.72** | **5.3** | **0.03** |
| **-Zn 1.0 wt.%** | **Inventive Example 13** | | **1Zn-0.5Mn-0.5Sr-0.5Cα** | **ZX105** | **289.5** | **0.85** | **2.2** | **0.02** |
| **- Mn 0.5 wt.%** | **Inventive Example 14** | | **1Zn-0.5Mn-0.5Sr-1Ca** | **ZX11** | **285.3** | **1.04** | **15.1** | **0.03** |
| **-Sr0.5wt.%** | **Comparative Example 11** | | **1Zn-0.5Mn-0.5Sr-2Cα** | **ZX12** | **282.5** | **2.37** | **13.8** | **0.48** |

**[Table 3]**

| | | **Alloy** | | | **Properties** | | **Standard Deviation (SD)** | |
|---|---|---|---|---|---|---|---|---|
| | | **Alloy** | **Chemical Composition (wt.%)** | **Code** | **UCS (MPα)** | **CR (mm/y)** | **UCS (MPα)** | **CR (mm/y)** |
| **Mn Effect** | **Inventive Example 15** | **ZMJX1x0505** | **1Zn-0Mn-0.5Sr-0.5Cα** | **ZMX1005** | **305.3** | **1.03** | **1.7** | **0.09** |
| **-Zn 1 wt.%** | **Inventive Example 13** | | **1Zn-0.5Mn-0.5Sr-0.5Cα** | **ZMX10505** | **289.5** | **0.85** | **2.2** | **0.02** |
| **- Mn 0.5 wt.%** | | | | | | | | |
| **- Ca 0.5 wt.%** | **Inventive Example 16** | | **1Zn-1Mn-0.5Sr-0.5Cα** | **ZMX1105** | **302.6** | **1.46** | **4.5** | **0.11** |
| **Sr Effect** | **Inventive Example 17** | **ZMJX105x05** | **1Zn-0.5Mn-0Sr-0.5Cα** | **ZJX1005** | **209.0** | **0.69** | **6.5** | **0.02** |
| **-Zn 1 wt.%** | **Inventive** | | **1Zn-0.5Mn-0.5Sr-0.5Cα** | **ZJX10505** | **289.5** | **0.85** | **2.2** | **0.02** |
| **- Sr 0.5 wt.%** | **Example 13** | | | | | | | |
| **- Ca 0.5 wt.%** | **Inventive Example 16** | | **1Zn-0.5Mn-1Sr-0.5Cα** | **ZJX1105** | **302.8** | **1.81** | **4.9** | **0.05** |
| **Ca Effect** | **Inventive Example 01** | **ZMJX050505x** | **0.5Zn-0.5Mn-0.5Sr-0Cα** | **ZX050** | **246.3** | **0.51** | **3.1** | **0.07** |
| **- Zn 0.5 wt.%** | **Inventive Example 19** | | **0.5Zn-0.5Mn-0.5Sr-0.5Cα** | **ZX0505** | **286.9** | **0.78** | **5.1** | **0.04** |
| **- Mn 0.5 wt.%** | | | | | | | | |
| **- Sr 0.5 wt.%** | **Inventive Example 20** | | **0.5Zn-0.5Mn-0.5Sr-1Cα** | **ZX051** | **291.4** | **0.83** | **5.9** | **0.04** |
| **Mn Effect** | **Comparative Example 12** | **ZMJXx050505** | **0Zn-0.5Mn-0.5Sr-0.5Cα** | **ZX005** | **246.8** | **18.17** | **3.4** | **2.24** |
| **- Mn 0.5 wt.%** | **Inventive Example 19** | | **0.5Zn-0.5Mn-0.5Sr-0.5Ca** | **ZX0505** | **286.9** | **0.78** | **5.1** | **0.04** |
| **- Sr 0.5 wt.%** | | | | | | | | |
| **- Ca 0.5 wt.%** | **Inventive Example 13** | | **1Zn-0.5Mn-0.5Sr-0.5Ca** | **ZX105** | **289.5** | **0.85** | **2.2** | **0.02** |
| **Zn Effect** | **Comparative Example 13** | **ZMJXx05051** | **0In-0.5Mn-0.5Sr-1Cα** | **ZX01** | **241.7** | **33.02** | **10.23** | **0.49** |
| **- Mn 0.5 wt.%** | **Inventive Example 20** | | **0.5Zn-0.5Mn-0.5Sr-1Ca** | **ZX051** | **291.0** | **0.83** | **5.9** | **0.04** |
| **- Sr 0.5 wt.%** | **Inventive Example 14** | | **1Zn-0.5Mn-0.5Sr-1Cα** | **ZX11** | **285.3** | **1.04** | **15.1** | **0.03** |
| **- Ca 1.0 wt.%** | **Inventive Example 21** | | **2Zn-0.5Mn-0.5Sr-1Cα** | **ZX21** | **294.2** | **1.57** | **4.32** | **0.25** |

Fig.1 is a graph illustrating the compressive strengths and corrosion rates of the Mg alloys according to inventive examples and comparative examples of the present disclosure.

Fig.2a is a graph illustrating the effect of Zn content on compressive strengths and corrosion rates of the Mg alloys. Fig.2b is a photograph illustrating microstructures of as-cast Mg alloys which demonstrates the effect of Zn content on compressive strengths and corrosion rates of the Mg alloys.

As shown in Figs.1 and 2a, the compressive strength tended to increase according to increase in Zn content. Nonetheless, when the Zn content is 3.0 wt.% or more, the corrosion rate may rapidly increase. Therefore, the Zn content may be preferably less than 3.0 wt.%, more preferably 2.0 wt.% or less, and even more preferably 1.5 wt.% or less. Especially, when the Zn content is 1.5 wt.% or less, the corrosion rate of the Mg alloy may be maintained at less than 1.0 mm/y. While Zn may contribute to the formation of passive protection films to improve corrosion features, corrosion rate may dramatically increase due to formation of a large amount of secondary phases with Zn content of 3.0 wt.% or more as shown in Fig.2b.

Fig.3a is a graph illustrating the effect of Mn content on the compressive strengths and corrosion rates of the Mg alloys with Zn content of 3.0 wt.%. Fig.3b is a graph illustrating effect of Mn content on compressive strengths and corrosion rates of the Mg alloy with Zn in the amount of 1.0 wt.%.

As shown Figs.l, 3a and 3b, compressive strength tended to increase according increase in Mn content, but differently based on the Zn content. That is, in the case of a Mg alloy comprising Zn in the amount of 3.0 wt.%, corrosion resistance increased according to increase in the Mn content and compressive strength increased when Mn is added in the amount of at least 1.0 wt.%. In the case of a Mg alloy comprising Zn in the amount of 1.0 wt.%, change in the corrosion rate according to increase in the Mn content was insignificant. The corrosion rate was maintained at less than 1.0 mm/y and the compressive strength increased by about 5 to 10%.

Fig.4a is a graph illustrating the effect of Sr content on the compressive strengths and corrosion rates of the Mg alloys with Zn content of 3.0 wt.%. Fig.4b is a graph illustrating the effect of Sr content on the compressive strengths and corrosion rates of the Mg alloys with Zn in the amount of 1.0 wt.%.

As shown in Figs. 1, 4a and 4b, the compressive strength showed a tendency to increase with increasing Sr content, but the extent of trend depended on the Zn content. That is, in the case of a Mg alloy comprising Zn in the amount of 3.0 wt.%, the change in corrosion rate according to increase in the Sr content was insignificant. Addition of Sr in the amount of 1.0 wt.% or more caused increase in compressive strength. In the case of a Mg alloy comprising Zn in the amount of 1.0 wt.%, compressive strength tended to rapidly increase up to the point of addition of Sr in the amount of 0.5 wt.% and then somewhat decrease. Corrosion rate tended to decrease to 0.7 mm/y up to the point of addition of Sr in the amount of 0.5 wt.% and then rapidly increase, but it remained below 2.0 mm/y.

Fig.5a is a graph illustrating the effect of Ca content on the compressive strengths and corrosion rates of the Mg alloys with Zn content of 1.0 wt.%. Fig.5b is a graph illustrating effect of Ca content on compressive strengths and corrosion rates of the Mg alloy with Zn content of 0.5 wt.%.

As shown in Figs. 1, 5a and 5b, when Zn content is relatively low, compressive strength tended to increase according to increase in Ca content. That is, in the case of a Mg alloy comprising Zn in the amount of 1.0 wt.%, improvement in compressive strength according to increase in Ca content was insignificant. In the aspect of controlling corrosion rate, Ca content of less than 2.0 wt.% is preferable. When Ca content was 1.0 wt.%, the corrosion rate was remained below 1.0 mm/y. In the case of a Mg alloy comprising Zn in the amount of 0.5 wt.%, compressive strength rapidly increased according to increase in Ca content while the effect on corrosion rate was insignificant. The corrosion rate was maintained at 0.8 mm/y.

Fig.6a is a graph illustrating the effect of Zn content on the compressive strengths and corrosion rates of the Mg alloys with Ca content of 0.5 wt.%. Fig.6b is a graph illustrating effects of Zn contents on compressive strengths and corrosion rates of the Mg alloys with Ca content of 1.0 wt.%.

As shown in Figs. 1, 6a and 6b, compressive strength showed a tendency to increase with increasing Zn content, but the extent of the trend depended on the Ca content. That is, in the case of a Mg alloy comprising Ca in the amount of 0.5 wt.%, the compressive strength increased according to increase in Zn content. Corrosion rate dramatically decreased by addition of Zn. Without the addition of Zn, rapid corrosion behavior occurs, and the corrosion rate decreased with Zn addition. This is because Zn contributes to the formation of passive protection film, thereby improving corrosion features. Meanwhile, in the case of a Mg alloy comprising Ca in the amount of 1.0 wt.%, the compressive strength increased and corrosion rate decreased with Zn addition. Corrosion rate tended to increase according to increase in the amount of Zn added. The lowest corrosion rate was shown when Zn was added in the amount of 0.5 wt.%.

Fig.7 is a graph illustrating the effect of Mn content on the compressive strengths and corrosion rates of the Mg alloys with Ca in the amount of 0.5 wt.%. As shown in Fig.7, improvement in compressive strength according to increase in the amount of Mn was insignificant. Corrosion rate tended to increase by addition in the amount of 1.0 wt.%.

Fig.8 is a graph illustrating the effect of Sr content on the compressive strengths and corrosion rates of the Mg alloys with Ca in the amount of 0.5 wt.%. As shown in Fig.8, according to increase in the Sr content, compressive strength rapidly increased and corrosion rate also increased. Specifically, when Sr was added in the amount of 1.0 wt.%, the corrosion rate rapidly increased but remained at 2.0 mm/y or below.

Fig.9 is a graph illustrating the compressive strengths and corrosion rates of the Mg alloys according to their component contents. As shown in Fig.9, in order to satisfy the target conditions of compressive strength of at least 250 MPa and corrosion rate of 2.0 mm/y or less, it may be preferable that the sum of the amount of Zn and the amount of Ca is at least 0.5 wt.% and at most 3.0 wt.% based on the total weight of the Mg alloy. Furthermore, as shown in Fig.9, in order to satisfy the target condition of a best mode of compressive strength of at least 250 MPa and corrosion rate of 1.0 mm/y or less, amount of Zn may be preferably at least 0.5 wt.% and less than 2.0 wt.% based on the total weight of the Mg alloy.

Fig.10a is a graph illustrating the compressive strengths of Mg alloys according to inventive examples and comparative examples of the present disclosure after 8 weeks of immersion. As shown in Fig.10a, after being immersed for 8 weeks, there were 5 species of Mg alloys (Z1^{∗}, ZMJ115, ZX101, ZX0505, ZX051 (Z1*=ZM105=ZX10=ZMJ10505)) with at least 86% of compressive strength. There were 9 species of Mg alloys (Z1, Z15, ZM10, ZMJ115, ZX101, ZX11, ZX12, ZX0505, ZX051) with at least 64% of compressive strength. There were 10 species of Mg alloys (Z1, Z15, ZM10, ZMJ115, ZX105*, ZX101, ZX11, ZX12, ZX0505, ZX051 (ZX105*=ZJX10505)) with at least 28% of compressive strength.

Fig.10b is a graph illustrating the compressive strengths of Mg alloys according to inventive examples and comparative examples of the present disclosure after 16 weeks of immersion. Among the alloys with compressive strength of at least 250MPa and corrosion rate of 2.0 mm/y or less after 1 week of immersion, 5 species (Z1, ZM115, ZX101, ZX0505, ZX051) with 86% of compressive strengths after 8 weeks of immersion were tested for immersion test for 16 weeks. Immersion tests on compression specimens were performed in PBS solution without replacement at 36.5°C for both 8-week and 16-week immersion tests. After removal of corrosion products, the compression tests at room temperature were performed. The compressive strength ratios after 16 weeks of immersion were compared with those of commercial alloys (WE43, XZ51) as a comparative group. As shown in Fig. 10b, after 16 weeks of immersion, all five species of ZMJX alloys according to the present disclosure demonstrated high compressive strength ratios of at least 90%, which are almost similar to those of 8-week immersions. The performance was similar to that of a commercial alloy, WE43. While WE43, the comparative group, demonstrated a high compressive strength ratio after 16 weeks, XZ51 was completely degraded after 8 weeks.

Fig.11 is a graph illustrating measurements of thermodynamics-based secondary phase fractions based on Zn content. Using thermodynamics DB system FactSage 8.0, secondary phase fraction with Scheil-Gulliver cooling standards was calculated. When Zn content in the alloy of Mg-xZn-0.5Mn-0.5Sr changed to 0.0, 0.1, 0.5, 1.0, 1.5, 2.0, 3.0, 4.0, 5.0 wt.%, the results are shown below by analyzing the effect of Zn by calculating changes in the secondary phase fraction and the total amount.
i) Zn < 2.0 wt.%
   The binary phase (Mg₁₇Sr₂) and ternary phase (SrZnMg) coexisted while the fraction of the secondary phase was maintained at less than 2.0 vol.%.
ii) Zn ≥ 2.0 wt.%

Only ternary phase (SrZnMg) appeared and the fraction of the secondary phase rapidly increased according to increase in Zn content.

Fig. 12 is a graph illustrating measurements of thermodynamics-based secondary phase fractions according to the Mn contents. Using the thermodynamics DB system FactSage 8.0, secondary phase fraction with Scheil-Gulliver cooling standards was calculated. Secondary phase fractions are calculated for 6 types of alloys within the range of target physical properties when the amount of Mn exceeds 0.5 wt.% and the results are shown below.
i) In the case where the amount of Mn was 0.5 wt.% and 1.5 wt.%, there was no change in the secondary phase fraction regardless of the target alloy (see dotted line).
ii) When Mn was added in the amount of 2.5 wt.%, insoluble Mn phase was formed, and thus the total summation of the secondary phase fractions was increased.
iii) In the equilibrium state, as the solid solubility limit of Mn is 2.2 wt.%, addition of other elements to the solution with Mn in the amount of 2.0 wt.% also increased the secondary phase fraction.

Figs. 13 to 16 are photographic images illustrating the exterior appearances of corrosion specimens after immersion tests of the as-cast Mg alloys according to inventive examples and comparative examples of the present disclosure. Figs. 17 to 21 are photographic images illustrating the microstructures of the as-cast Mg alloys according to inventive examples and comparative examples of the present disclosure.

Although the present disclosure has been described in detail with reference to specific exemplary embodiments, those skilled in the art with common knowledge will appreciate that various modifications and variations within the scope of the present disclosure are possible through additions, modifications, deletions, etc. of the components and that these modifications and variations also fall within the scope of the claims of the present disclosure.

## Claims

1. A biodegradable Mg alloy comprising,
Zn in the amount of at least 0.5 wt.% and less than 3.0 wt.%;
Mn in the amount of at least 0.0 wt.% and at most 2.0 wt.%;
Sr in the amount of at least 0.0 wt.% and less than 3.0 wt.%;
Ca in the amount of at least 0.0 wt.% and at most 2.0 wt.%; and
Mg in the rest amount, based on the total weight of the Mg alloy.

2. The biodegradable Mg alloy according to claim 1, wherein the sum of the amount of Zn and the amount of Ca is at least 0.5 wt.% and at most 3.0 wt.%, based on the total weight of the Mg alloy.

3. The biodegradable Mg alloy according to claim 2, wherein the amount of Zn is at least 0.5 wt.% and less than 2.0 wt.%, based on the total weight of the Mg alloy.

4. The biodegradable Mg alloy according to claim 1, wherein the amount of Sr is at least 0.0 wt.% and at most 2.0 wt.% and the amount of Ca is at least 0.0 wt.% and less than 2.0 wt.%, based on the total weight of the Mg alloy.

5. The biodegradable Mg alloy according to claim 1, wherein the corrosion resistance of the Mg alloy is improved by suppressing micro-galvanic corrosion between a matrix and impurities or secondary phases and by improving passivation state features of a film formed on surface.

6. The biodegradable Mg alloy according to claim 1, wherein the compressive strength of the biodegradable Mg alloy is at least 250 MPa in the as-cast state.

7. The biodegradable Mg alloy according to claim 1, wherein the corrosion rate of the biodegradable Mg alloy is 2.0 mm/y or less in the as-cast state.

8. The biodegradable Mg alloy according to claim 1, wherein, after 8 weeks of immersion in phosphate-buffered saline (PBS) solution, the Mg alloy still has more than 0% of the compressive strength in the as-cast state.

9. A biodegradable implant consisted of the biodegradable Mg alloy according to any one of claims 1 to 8.

10. The biodegradable implant according to claim 9, wherein the implant is an orthopedic device, a parietal facial device or a cardiovascular device.

11. A method of manufacturing a biodegradable implant, the method comprising the steps of;
i) preparing molten mixture by melting components of Zn in the amount of at least 0.5 wt.% and less than 3.0 wt.%; Mn in the amount of at least 0.0 wt.% and at most 2.0 wt.%; Sr in the amount of at least 0.0 wt.% and less than 3.0 wt.%; Ca in the amount of at least 0.0 wt.% and at most 2.0 wt.%; and Mg in the rest amount, based on the total weight of the Mg alloy, together; and
ii) casting the molten mixture of step i) to obtain the implant.

12. The method of manufacturing a biodegradable implant according to claim 11, wherein, in step i), the sum of the amount of Zn and the amount of Ca is at least 0.5 wt.% and at most 3.0 wt.%, based on the total weight of the Mg alloy.

13. The method of manufacturing a biodegradable implant according to claim 11, wherein, in step i), the amount of Sr is at least 0.0 wt.% and at most 2.0 wt.% and the amount of Ca is at least 0.0 wt.% and less than 2.0 wt.%, based on the total weight of the Mg alloy.

14. The method of manufacturing a biodegradable implant according to claim 11, wherein the compressive strength of the prepared biodegradable Mg alloy is at least 250 MPa in the as-cast state.

15. The method of manufacturing a biodegradable implant according to claim 11, wherein the corrosion rate of the prepared biodegradable Mg alloy is 2.0 mm/y or less in the as-cast state.
